# EUROPEAN PATENT SPECIFICATION

(11) **EP 2 040 669 B1**
(45) Date of publication and mention of the grant of the patent: **26.08.2009**
(21) Application number: 07712193.7
(22) Date of filing: 08.02.2007
(51) Int. Cl.: A61K 8/98, A61K 35/64, A61P 17/02

(54) **WOUND CARE TREATMENT PRODUCT**
WUNDBEHANDLUNGSPRODUKT
PRODUIT DE SOINS POUR BLESSURES

(30) Priority: 01.06.2006 BE 200600306
(43) Date of publication of application: 01.04.2009
(73) Proprietor: Sano Medical BVBA, 8490 Varsenare (BE)
(72) Inventor: VANDEPUTTE, Jan, 8490 Varsenare (BE)
(74) Representative: Ostyn, Frans
(86) International application number: PCT/EP2007/051233
(87) International publication number: WO 2007/137881

(56) References cited:
- WO-A-20/04093569
- WO-A-20/06021924

## Description

The invention relates to a wound care treatment product on the basis of honey.

Relatively few biotechnology products have been developed for treating wounds and the treatment of acute wounds such as partial-thickness bums. Most of the efforts have been directed towards chronic wounds, which do require specific and sufficient amounts of cellular growth factors for healing. The most conventional option of chronic ulcer treatment involves sharp debridement to remove all nonviable tissue (operative, enzymatic, etc.), a non-weight-bearing regimen, moist saline dressings changed once a day or less at which times the skin around the ulcers are cleansed with mild soap and water. Current advanced treatment for chronic ulcers include growth factors, skin replacement therapy, enzymatic and mechanical debridement to clean ischemic tissue, moist wound dressings, silver and honey tissues, non-antibiotic cleansers, and antibiotics (Edmonds *et al.,* 2000, Lipsky and Berendt 2000, Moulin *et al.,* 1998, Mandracchia *et al.,* 2001). However, current therapy for chronic wounds is rather ineffective. In fact, Regranex TM gel or Becaplermin (recombinant-human platelet-derived growth factor-BB), the only biological product in the market for chronic wounds (diabetic neuropathy ulcers) has shown only 9-23% improvement over placebo and 4-22% improvement over good ulcer care alone (Mandracchia *et al.,* 2001, Edmonds *et al.,* 2000, Wieman 1998). Thus, an effective treatment for wounds, chronic and/or acute, is needed.

In wound care, honey has been used for thousands of years. The Egyptian hieroglyphs translations were the first described combinations of honey, fat or oil that were found. The major advantage of honey in wound care is the high osmotic activity that accelerates debridement of necrotic tissue and procures the antibacterial effect. Other activities of honey in wound care are more hypothetic and not yet fully searched or understood. There are a couple of patents with honey for wound healing that can be found, f.i. US 6,174,535, US 5,980,875, WO 02/30467, WO2004/000339 (claiming priority of BE 1014976).

These patents concentrate on how to define the honey, such as the amount of hydrogen peroxide it produces and other parameters. Also in NL 1016398, it is specifically stated that honey must be irradiated for the treatment of wounds. Honey contains a number of bacteria, yeasts and fungi. Some of those bacteria are anaerobic such as *Clostridium Botulinum* strains. These can usually be found in raw honey and can, when used on wounds, develop and cause gas gangrene. For instance in 270 samples of unprocessed honey from seven different countries, 8,5 % carried up to 40 - 80 spores per gram of honey (Postmes T., van den Bogaard A.E., Hazen M., The sterilization of honey with cobalt 60 gamma radiation : a study of honey spiked with spores of Clostridium Botilinum and Bacillus Subtilis, Experientia 51 (1995), p. 986 - 989). Therefore, honey that is used to make a wound care ointment or gel is irradiated with an average dose of 15 -30 kGry. The irradiation of honey with such high doses is only done for the last 5 years and when one measures the enzyme activity (glucose oxidase) before and after the irradiation, then it is clear that most of the honeys have lost all the active enzymes making the honey no longer producing hydrogen peroxide. Some honeys are a bit more resistant against radiation (most of the time those ones having a high flavenoids content), but are then forming so much gas during the irradiation process that barrels are bursting open or flow over. During the irradiation, the honey becomes like a white cream with lots of foam, and it takes several weeks before the honey starts to get its normal colour and consistency. It is also our experience (by doing the accelerated aging test with irradiated honey and honey based wound care ointments) that this irradiated honey continues to form and produce gas for several years. This causes problems for the shelf live of the wound care ointment made with this irradiated honey. Furthermore, the produced gas behaves as a kind of propellant that, when a tube is opened, the ointment / gel rapidly comes out of the tube; wherein in a number of cases the tube completely empties. It is also our experience that ointments / gels which are made with (on the basis of) irradiated honey seems to become brown over a period of one year (in closed aluminium tubes), indicating that a caramellisation process is induced by the irradiation of the honey. This process of caramellisation can easily be accelerated by exposing the ointment / gel at higher temperatures. When honey ointment tubes are exposed to temperatures above 28 - 35 °C for a period of two months, the appearance of the ointment is completely changed from a yellow white cream like product to a sticky dark brown crusty syrup. Some of the tubes even exploded during the test (within 2 months). It can consequently be concluded that the standard irradiation of honey for use in wound care is actually destroying the honey and compromises the function /activity of the honey.

From WO 2006/021924, it is known to use ozonated avocado oil in its pure form for wound care purposes.

Ozone (O₃) is the triatomic form of oxygen (O₂) and is, as a very reactive gas, difficult to stabilize for long periods of time in a useable form. It is used as an antibacterial agent in many industrial processes usually in sterilizing water or surfaces where food stuff, f.i. raw meat, is processed. This is usually done be bubbling generated ozone gas through the medium. Ozone is a powerful oxidizer that is capable of efficiently killing or destroying a wide variety of viruses (including HIV), bacteria (it kills E-coli 3125 times faster than chlorine, and it converts into ordinary oxygen in the process, leaving no chemical residuals behind), fungi, yeasts and other toxins. It also oxidizes phenolics, pesticides, detergents, chemical manufacturing wastes and aromatic compounds (such as smelly compounds usually formed in cancer wounds) more rapidly and effectively than chlorine, yet without its harmful residues (Chemical Technology An Encyclopaedic Treatment, Vol 1, New York: Barnes & Noble Inc., 1968, p 82-83). For this reason, ozone has become the first choice to disinfect and purify drinking water and waste water through a wide variety of applications. The Encyclopedia of Chemical Technology (page 704) reports that ozone displays an all or nothing effect in terms of destroying bacteria. This effect can be attributed to the high oxidation potential of ozone. Ozone is such a strong germicide that only a few micrograms per liter are required to measure germicidal action. Shortly after patenting his first ozone generator, in 1890 Nikola Tesla began marketing an ozonated olive oil for medical applications. Nikola Tesla created his ozonated oil by bubbling ozone through pure olive oil in the presence of a magnetic field for eight weeks. From 1904, ozonated olive oil, also known as Glycozone, began appearing in medical literature, such as "The Medical Uses of Hydrozone and Glycozone", 9th Edition, by New York Chemist Charles Marchland.

However, by bubbling ozone through an ozone resistant container (such as a glass container), the ozone gas is trapped, and begins to react with the oil. In essence, what is occurring is a catalytic reaction that actually burns the olive oil. One of the resultant compounds is C₁₀H₁₈O₃, with the hydrogen and carbon complex. Some of the terpene gas remains trapped within the oil, and the rest is released into the environment.

While some people may believe that fully ozonated olive oil is an ozone carrier, the oxygen is actually bound and released as a peroxide (O-O-H bond). Ozonated olive oil will hold actual ozone gas for a limited amount of time, but in its "free form" state. The ozone gas is rapidly released from the oil when the oil gets a temperature of 25 °C or higher, or comes into contact with water or water vapour.

The average time in order to saturate oil with ozon is, dependant on the type of ozone generator one is using, about 3 weeks. Once olive oil is completely ozonated, it will turn into a nearly clear, gel substance. The smell of ozone being emitted from the olive oil will be noticeable. The final product must be kept refrigerated at all times. When the olive oil is kept below 15°C, it will keep the ozone for a longer period of time even over 10 years.

Ozonated oil is actually created by a redox reaction. The ozone literally burns the oil, and three primary, organic peroxides are created throughout the entire process. In other words, the first peroxide created reacts a second time to produce a second peroxide, and then finally once again to form C₁₀H₁₈O₃. The final process is quite noticeable as the entire substance will turn into a white foam. Once this white foam settles, ozonating any further is pointless, as the original oil is no longer present, and the compounds have been taken to a state that no longer reacts with ozone.

According to research conducted by Hulda Clark, ozonated olive oil may be utilized anywhere from as little of 20 minutes to 12 hours of ozonation. Clark recommends the use of partially ozonated olive oil for internal use, as a part of a liver cleansing program and in order to remove PCB's from the body (by taking 2 tablespoons of partially ozonated olive oil three times a day for 2 - 3 weeks).

The purpose of the invention is on the one hand to provide a wound care treatment product on the basis of honey, in which the micro-organisms in the honey are diminished so that it safely can be used in wound care.

The purpose of the invention is solved by providing a wound care treatment product on the basis of honey, wherein the honey is a raw, unprocessed honey and wherein the honey furthermore comprises ozonated oil.

In an advantageous wound care treatment product according to the invention, the ozonated oil is an oil wherein ozone gas has been administered till a minimum of 15 mg ozone/gram oil is reached.

In a favourable embodiment of a wound care treatment product according to the invention, the ozonated oil comprises maximum 53 mg of ozone per gram oil.

In a preferred embodiment of a wound care treatment product according to the invention, the ozonated oil is a vegetable oil such as olive oil, sunflower oil, coco oil, etc.

In a preferred wound care treatment product according to any the invention, the wound care treatment product comprises between 5 to 50 w/w % ozonated oil with relation to the total composition of the wound care treatment product.

An advantageous wound care treatment product according to the invention comprises one or more ingredients selected from a PEG compound, CMC (natrium-carboxy-methyl-cellulose), propylene glycol, Aloe vera, lanolin, cod liver oil, calendula oil, glucose oxidase, superoxide dismutase (SOD), lactoperoxidase (LP), methyl sulphonyl methane (MSM) and Lactoferrin (LF).

In a preferred embodiment of a wound care treatment product according to the invention, the wound care treatment product comprises one or more polymers selected from the group consisting of vinyl polymer, polysaccharide polymer, glyeosaminoglycan polymer, protein polymer, polyoxyethylenepolyoxypropylene polymer and acryl amide polymer.

In a more preferred embodiment of a wound care treatment product according to the invention, the concentration of polymer(s) is about 0,5 w/w % to about 60,0 w/w % with relation to the total composition of the wound care treatment product and the average molecular weight of the polymer(s) is of about 500 to about 13,000,000.

In a favourable wound care treatment product according to the invention, the wound care treatment product comprises a lactoferrin concentration of about 0,0001 - 30 % w/w % with relation to the total composition of the wound care treatment product.

In a preferred embodiment of a wound care treatment product according to the invention, the wound care treatment product is
- a local applicable product such as a gel, an ointment, a cream, or
- a carrier consisting of a gauze, a polyester or a polypropylene net onto which a gel, an ointment or a cream is applied resulting in a fatty gauze, a hydrocellular (polyurethane) foam plate wherein a gel / ointment / cream is impregnated, a hydrogel wherein a gel / ointment / cream is incorporated, or an alginate that is mixed with a gel / ointment / crème, or
- any other standard wound treatment product or healing therapy.

In order to further clarify the properties of the present invention and to point out its advantages and particulars, a more detailed description of a wound care treatment product according to the invention will now follow. It may be obvious that in the following description, nothing may be interpreted as being a restriction of the protection of the device according to the invention, demanded for in the claims.

On the other hand, this invention will be further illustrated by the examples, which should also be considered as being not limiting to the scope of the invention as such and as expressed in the following claims.

The wound care treatment product according to the invention comprises a combination of a raw, unheated (not heated above 38°C), unprocessed honey and an ozonated oil. The honey can indeed be cold extracted, because this does not change anything at all to the composition of the honey. Also a minimal radiation of the honey is allowed, to a maximum of 5 kGy, on the condition that the enzymatic activity of the honey is not changed substantially.

The ozonated oil is preferably a vegetable oil where ozone gas has been administered till a minimum of 15 mg ozone/gram oil is reached. The amount of ozonated oil should at least be 5 w/w % of the total composition of the wound care treatment product and can add up to 50 w/w % of the total composition of the wound care treatment product. The ozonated oil is a vegetable oil such a a.o. olive oil, sunflower oil, coconut oil.

Tests have been done to measure the effectiveness of the ozonated oil in combination of raw, unirradiated honey. Average raw unirradiated bio honey contains 250 - 400 colony forming units per gram micro-organisms (total count, aerobic/anaerobic bacteria, fungi and yeasts). When this oil is mixed with 20% ozonated oil, the total count of micro-organisms drops to 10 - 70 colony forming units per gram. The anaerobic bacteria including spores are also killed by the ozone.

The European pharmacopoeia states that a wound care product such as ointment /gel may contain no more that 100 micro-organisms (colony forming units per gram) and should be free of wound pathogens such as *Staphylococcus aureus , Pseudomonas aeruginosa ,* .... The last (pathogen bacteria) are usually not found in honey.

The wound care treatment product can comprise one or more ingredients selected from a PEG compound (Magrocol flakes) , propylene glycol, Aloe vera, lanolin, cod liver oil, calendula oil, glucose oxidase, superoxide dismutase (SOD), lactoperoxidase (LP), methyl sulphonyl methane (MSM) and Lactoferrin (LF).

Furthermore, the wound care treatment product can comprise one or polymers selected from the group consisting of vinyl polymer (f.i. polyacrylic acid, polymethacrylic acid, polyvinyl pyrrolidone and polyvinyl alcohol), polysaccharide polymer (f.i. cellulose, cellulose derivatives, glycosaminoglycans, agar, pectin, alginic acid, dextran, starch, and chitosan), glyeosaminoglycan polymer (f.i. hyaluronic acid, chondroitin, chondroitin-4-sulfate, chondroitin-6-sulfate, dermatan sulfate, keratan sulfate, heparin sulfate and heparin), protein polymer (f.i. collagen, gelatin and fibronectin), polyoxyethylene-polyoxypropylene polymer (f.i. polyoxyethylenepolyoxypropylene block copolymer) and acryl amide polymer (f.i. polyacrylamide or polymethacrylamide). Preferably, the polyoxyethylenepolyoxypropylene block copolymer is F88 or F127.

Preferably, the concentration of polymer(s) is about 0,5 w/w % to about 60,0 w/w % with relation to the total composition of the wound care treatment product, and the average molecular weight of the polymer(s) is of about 500 to about 13,000,000.

Furthermore, the wound care treatment product can comprise a lactoferrin concentration (lactoferrin or N-terminallactoferrin variant in which at least the N-terminal glycine residue is truncated or substituted) within the range of about 0,0001 w/w % to about 30 w/w % of the total composition of the wound care treatment product.

The wound care treatment product can be a product that is locally applicable such as a gel, an ointment, a cream, or can be a carrier consisting of a gauze, a polyester or a polypropylene gauze onto which a gel, an ointment or a cream is applied resulting in a fatty gauze, a hydrocellular (polyurethane) foam plate wherein a gel /ointment / cream is impregnated, a hydrogel wherein a gel / ointment / cream is incorporated, or an alginate that is mixed with a gel / ointment / crème, or can be any other standard wound treatment product or healing therapy.

### Examples of wound care treatment products

### Example 1 :

An aqueous gel having a viscosity in the range of about 1 to about 12,000,000 cP at room temperature, together with an amount of recombinant human lactoferrin (rhLF lactoferrin or an N-terminallactoferrin variant thereof, such that at least the N-terminal glycine residue is truncated or substituted), that is sufficient to obtain an improvement in the wound healing, and a polymer selected from the list as mentioned above, and having a concentration and an average molecular weight as mentioned above.

### Example 2 :

An aqueous gel (gel on the basis of water) having a viscosity in the range of about 1 to about 12,000,000 cP at room temperature, together with an amount of a lactoferrin that is sufficient to provide an improvement in the wound healing, in a pharmaceutically acceptable polymer selected from the group as mentioned above, and having a concentration and an average molecular weight as mentioned above.

### Example 3 :

A third example of a wound care product is a pharmaceutically acceptable carrier wherein the wound care treatment product comprises a lactoferrin in an amount sufficient to provide an improvement in the wound healing together with the honey and the ozonated oil.

### Example 4 :

Ointment / gel / cream consisting of
15,3% ozonated oil
60% raw honey
12% PEG 3500
12% Propylene glycol
0,5% Vit C
0,05% Vit E
0,05 % Glucose oxidase
0,1 % Lactoperoxidase

### Example 5 :

Ointment / gel / cream consisting of
35% ozonated oil
35% raw honey
17% PEG 4000
12,3% Propylene glycol
0,5% Vit C
0,05% Vit E
0,05 % Glucose oxidase
0,1% lactoferrine

### Example 6 :

Ointment / gel / cream consisting of
10% ozonated oil
40% raw honey
15% PEG 4000
34,1% Propyleneglycol
0,5% Vit C
0,25% Vit E
0,05 % Glucose oxidase
0,05 % Lactoperoxidase
0,05% lactoferrine

### Example 7:

Ointment / gel / cream consisting of
10% ozonated oil
40% raw honey
15% CMC (Natrium-carboxy-methyl-cellulose)
34,1 % Propylene glycol
0,5% Vit C
0,25% Vit E
0,05 % Glucose oxidase
0,1 % Lactoperoxidase

### Example 8:

Ointment / gel / cream consisting of
50% ozonated oil
25% raw honey
10% CMC (Natrium-carboxy-methyl-cellulose)
14,1% Propylene glycol
0,5% Vit C
0,25% Vit E
0,05 % Glucose oxidase
0,1 % Lactoperoxidase

### Example 9:

Ointment / gel / cream consisting of
5% ozonated oil
50% raw honey
15% CMC (Natrium-carboxy-methyl-cellulose)
29,1% Propylene glycol
0,5% Vit C
0,25% Vit E
0,05 % Glucose oxidase
0,1 % Lactoperoxidase

Concerning the examples 4 to 9, the ointment / gel / cream can furthermore be sprayed on a gauze, polyester or polypropylene net resulting in a fatty gauze, can be impregnated in a hydrocelluar foam (polyurethane) foam plate, can be incorporated in a hydrogel plate or can be mixed with an alginate; the amount of ointment / gel / crème being between 4- 10 g per 100 cm².

### Example 10:

As a carrier for the wound care treatment product according to the invention, the following materials can be used:
- Woven or non-woven gauze knitted net (standard use in wound care)
- Polyester net, multifilament knitted with a density of -130/m²
- Monofilament polypropylene knitted with a diameter of 0,14 mm to 0,20 mm
- A polyacetate carrier
- A foam with a :
   * thickness between 0,3 cm to 1,5 cm
   * with a density between 70 and 300 kg/m³
   * cell structure between 170 - 500 micrometers (um)
   * a fluid capacity after draining of 10-20 g water/g foam
   * a fluid capacity after compression between 10 -14 g water/g foam
   * a moisture vapour transfer capacity of 3500 - 4500 g/m²/24 hours
   * linear swell in length direction between 15 - 30 %
   * linear swell in height direction between 15 - 25%
   * ER/RR-dry % elongation between 200 - 450%
   * ER/RR-dry % strength at break between 100 - 300 kPa
   * Tear resistance between 2,5 - 8 kPa
   * Air permeability between 35 - 90 I/min/dm²

### Example 11:

A hydrogel plate can consist of:
5% ozonated oil
30,9% raw honey
30% polyacrylamide
30% glycerol
0,5% Vit C
0,5% Vit E
0,05% lactoperoxidase oxidase
0,05% glucose oxidase
3,0% lactoferrine

### Example 12:

A hydrogel plate can consist of:
5% ozonated oil

25 % raw honey
60 % polyacrylamide or amps
5 % water
4 % glycerol
1 % lactoferrine

Such standard wound care treatment products can be administered for at least one week, six weeks, 12 weeks, 36 weeks, etc. or any range in between.

## Claims

1. Wound care treatment product on the basis of honey, **characterised in that** the wound care treatment product comprises raw, non-heated honey and an ozonated oil.

2. Wound care treatment product according to claim 1, **characterised in that** the ozonated oil is an oil wherein ozone gas has been administered till a minimum of 15 mg ozone/gram oil is reached.

3. Wound care treatment product according to claim 1 or 2, **characterised in that** the ozonated oil comprises a maximum of 53 mg of ozone per gram oil.

4. Wound care treatment product according to any one of claims 1 to 3, **characterised in that** the ozonated oil is a vegetable oil such as amongst other olive oil, sunflower oil, coconut oil.

5. Wound care treatment product according to any one of claims 1 to 4, **characterised in that** the wound care treatment product comprises between 5 to 50 w/w % ozonated oil with relation to the total composition of the wound care treatment product.

6. Wound care treatment product according to any one of claims 1 to 5, **characterised in that** the wound care treatment product comprises with one or more ingredients selected from a PEG compound, CMC (natrium-carboxy-methyl-cellulose), propylene glycol, Aloe vera, lanolin, cod liver oil, calendula oil, glucose oxidase, superoxide dismutase (SOD), lactoperoxidase (LP), methyl sulphonyl methane (MSM) and Lactoferrin (LF).

7. Wound care treatment product according to any one of claims 1 to 6, **characterised in that** the wound care treatment product comprises one or more polymers selected from the group consisting of vinyl polymer, polysaccharide polymer, glycosaminoglycan polymer, protein polymer, polyoxyethylenepolyoxypropylene polymer and acryl amide polymer.

8. Wound care treatment product according to claim 7, **characterised in that** the concentration of polymer(s) is about 0,5 w/w % to about 60,0 w/w % of the total composition of the wound care treatment product and the average molecular weight of the polymer(s) is of about 500 to about 13,000,000.

9. Wound care treatment product according to any one of claims 1 to 8, **characterised in that** the wound care treatment product comprises a lactoferrin concentration of about 0,0001 - 30 % w/w % with relation to the total composition of the wound care treatment product.

10. Wound care treatment product according to any one of claims 1 to 9, **characterised in that** the wound care treatment product is
- a local applicable product such as a gel, an ointment, a cream, or
- a carrier consisting of a gauze, a polyester or a polypropylene net onto which a gel, an ointment or a cream is applied resulting in a fatty gauze, a hydrocellular (polyurethane) foam plate wherein a gel / ointment /cream is impregnated, a hydrogel sheet wherein a gel / ointment / cream is incorporated, or an alginate that is mixed with a gel / ointment / crème, or
- any other standard wound treatment product or healing therapy.

## Patentansprüche

1. Wundbehandlungsprodukt auf Basis von Honig, **dadurch gekennzeichnet, dass** das Wundbehandlungsprodukt rohen, nicht erhitzten Honig und ein ozonisiertes Öl enthält.

2. Wundbehandlungsprodukt nach Anspruch 1, **dadurch gekennzeichnet, dass** es sich bei dem ozonisierten Öl um ein Öl handelt, in welches Ozongas bis zur Erreichung eines Minimums von 15 mg Ozon/Gramm Öl eingeleitet wurde.

3. Wundbehandlungsprodukt nach Anspruch 1 oder 2, **dadurch gekennzeichnet, dass** das ozonisierte Öl maximal 53 mg Ozon pro Gramm Öl enthält.

4. Wundbehandlungsprodukt nach einem der Ansprüche 1 bis 3, **dadurch gekennzeichnet, dass** es sich bei dem ozonisierten Öl um eine Pflanzenöl, wie Olivenöl, Sonnenblumenöl, Kokosöl usw., handelt.

5. Wundbehandlungsprodukt nach einem der Ansprüche 1 bis 4, **dadurch gekennzeichnet, dass** das Wundbehandlungsprodukt zwischen 5 bis 50 Gew.-% ozonisiertes Öl, bezogen auf die Gesamtzusammensetzung des Wundbehandlungsprodukts, enthält.

6. Wundbehandlungsprodukt nach einem der Ansprüche 1 bis 5, **dadurch gekennzeichnet, dass** das Wundbehandlungsprodukt eine oder mehrere Inhaltsstoffe enthält, welcher/welche aus einer PEG-Verbindung, CMC (Natriumcarboxymethylcellulose), Propylenglycol, Aloe vera, Lanolin, Lebertran, Ringelblumenöl, Glucose-Oxidase, Superoxiddismutase (SOD), Lactoperoxidase (LP), Methylsulphonylmethan (MSM) und Lactoferrin (LF) ausgewählt ist/sind.

7. Wundbehandlungsprodukt nach einem der Ansprüche 1 bis 6, **dadurch gekennzeichnet, dass** das Wundbehandlungsprodukt ein oder mehrere Polymere enthält, das/die aus der Gruppe ausgewählt ist/sind, welche aus Vinylpolymer, Polysaccharid-Polymer, Glykosaminoglykan-Polymer, Protein-Polymer, Polyoxyethylen-Polyoxypropylen-Polymer und Acrylamid-Polymer besteht.

8. Wundbehandlungsprodukt nach Anspruch 7, **dadurch gekennzeichnet, dass** die Konzentration des Polymers beziehungsweise der Polymere etwa 0,5 Gew.-% bis etwa 60,0 Gew.-% der Gesamtzusammensetzung des Wundbehandlungsprodukts und das mittlere Molekulargewicht des Polymers bzw. der Polymere etwa 500 bis etwa 13.000.000 beträgt.

9. Wundbehandlungsprodukt nach einem der Ansprüche 1 bis 8, **dadurch gekennzeichnet, dass** die Lactoferrin-Konzentration in dem Wundbehandlungsprodukt etwa 0,0001 - 30 Gew.-%, bezogen auf die Gesamtzusammensetzung des Wundbehandlungsprodukts, beträgt.

10. Wundbehandlungsprodukt nach einem der Ansprüche 1 bis 9, **dadurch gekennzeichnet, dass** das Wundbehandlungsprodukt ein
- lokal applizierbares Produkt, wie etwa ein Gel, eine Salbe oder eine Creme, oder
- ein Träger, bestehend aus einem Gaze-, einem Polyester- oder einem Polypropylen-Netz, auf welches ein Gel, eine Salbe oder eine Creme appliziert wurde, wodurch eine Fettgaze erhalten wird, einer hydrozellulärer (Polyurethan-)Schaumstoffplatte, die mit einem Gel/einer Salbe/einer Creme imprägniert ist, einer Hydrogel-Folie, in die ein Gel/eine Salbe/eine Creme eingearbeitet ist, oder einem Alginat, das mit einem Gel/einer Salbe/einer Creme gemischt ist, oder
- ein beliebiges anderes standardmäßiges Wundbehandlungsprodukt oder Heilverfahren
ist.

## Revendications

1. Produit de traitement pour le soin des blessures à base de miel, **caractérisé en ce que** le produit de traitement pour le soin des blessures comprend du miel brut non chauffé et une huile ozonée.

2. Produit de traitement pour le soin des blessures selon la revendication 1, **caractérisé en ce que** l'huile ozonée est une huile dans laquelle de l'ozone gazeux a été administré jusqu'à ce qu'un minimum de 15 mg d'ozone/gramme d'huile soit atteint.

3. Produit de traitement pour le soin des blessures selon la revendication 1 ou 2, **caractérisé en ce que** l'huile ozonée comprend un maximum de 53 mg d'ozone par gramme d'huile.

4. Produit de traitement pour le soin des blessures selon l'une quelconque des revendications 1 à 3, **caractérisé en ce que** l'huile ozonée est une huile végétale telle que l'huile d'olive, l'huile de tournesol et l'huile de noix de coco, entre autres.

5. Produit de traitement pour le soin des blessures selon l'une quelconque des revendications 1 à 4, **caractérisé en ce que** le produit de traitement pour le soin des blessures comprend entre 5 et 50 % en poids d'huile ozonée par rapport à la composition totale du produit de traitement pour le soin des blessures.

6. Produit de traitement pour le soin des blessures selon l'une quelconque des revendications 1 à 5, **caractérisé en ce que** le produit de traitement pour le soin des blessures comprend un ou plusieurs ingrédients choisis parmi un composé de PEG, la CMC (carboxyméthylcellulose de sodium), le propylène glycol, l'aloe vera, la lanoline, l'huile de foie de morue, l'huile de calendula, la glucose oxydase, la superoxyde dismutase (SOD), la lactoperoxydase (LP), le méthyle sulphonyle méthane (MSM) et la lactoferrine (LF).

7. Produit de traitement pour le soin des blessures selon l'une quelconque des revendications 1 à 6, **caractérisé en ce que** le produit de traitement pour le soin des blessures comprend un ou plusieurs polymères choisis dans le groupe constitué par un polymère de vinyle, un polymère de polysaccharide, un polymère de glycosaminoglycane, un polymère de protéine, un polymère de polyoxyéthylènepolyoxypropylène et un polymère d'acrylamide.

8. Produit de traitement pour le soin des blessures selon la revendication 7, **caractérisé en ce que** la concentration du ou des polymères est d'environ 0,5 % en poids à environ 60,0 % en poids de la composition totale du produit de traitement pour le soin des blessures et le poids moléculaire moyen du ou des polymères est d'environ 500 à environ 13 000 000.

9. Produit de traitement pour le soin des blessures selon l'une quelconque des revendications 1 à 8, **caractérisé en ce que** le produit de traitement pour le soin des blessures comprend une concentration de lactoferrine d'environ 0,0001 à 30 % en poids par rapport à la composition totale du produit de traitement pour le soin des blessures.

10. Produit de traitement pour le soin des blessures selon l'une quelconque des revendications 1 à 9, **caractérisé en ce que** le produit de traitement pour le soin des blessures est
- un produit applicable local tel qu'un gel, un onguent, une crème, ou
- un support constitué d'une gaze, d'un filet en polyester ou en polypropylène sur lequel un gel, un onguent ou une crème est appliqué pour former une gaze grasse, une plaque de mousse hydrocellulaire (polyuréthanne) dans laquelle un gel/un onguent/une crème est imprégné, un film en hydrogel dans lequel un gel/un onguent/une crème est incorporé ou un alginate qui est mélangé avec un gel/un onguent/une crème, ou
- un autre produit de traitement des blessures ou une autre thérapie de guérison standard quelconque.
